# EUROPEAN PATENT SPECIFICATION

(11) **EP 1 709 421 B1**
(45) Date of publication and mention of the grant of the patent: **02.08.2017**
(21) Application number: 05705399.3
(22) Date of filing: 10.01.2005
(51) Int. Cl.: G01N 33/574

(54) **METHODS FOR DIAGNOSIS AND PRONOSIS OF CANCERS OF EPITHELIAL ORIGIN**
VERFAHREN ZUR DIAGNOSE UND PROGNOSE VON KREBSERKRANKUNGEN EPITHELIALER GENESE
METHODES DE DIAGNOSTIC ET DE PRONOSTIC DE CANCERS D'ORIGINE EPITHELIALE

(30) Priority: 09.01.2004 US 535306 P
(43) Date of publication of application: 11.10.2006
(73) Proprietor: CHILDREN'S MEDICAL CENTER CORPORATION, Boston, Massachusetts 02115 (US)
(72) Inventor: MOSES, Marsha, A., Brookline, MA 02445 (US); ROY, Roopali, Newton, MA 02459 (US)
(74) Representative: Brown, David Leslie
(86) International application number: PCT/US2005/000714
(87) International publication number: WO 2005/071387

(56) References cited:
- WO-A-97/40072
- WO-A-02/059377
- US-A1- 2002 081 641
- LE PABIC H. ET AL.: "ADAM12 in human liver cancers: TGF-b-regulated expression in stellate cells is associated with matrix remodeling" HEPATOLOGY, vol. 37, no. 5, May 2003 (2003-05), pages 1056-1066, XP002486182
- ROY R. ET AL.: 'ADAM 12 cleaves extracellular matrix proteins and correlates with cancer status and stage' THE JOURNAL OF BIOLOGICAL CHEMISTRY vol. 279, no. 49, 03 December 2004, pages 51323 - 51330, XP002321631
- FRÖHLICH C. ET AL.: "Molecular profiling of ADAM12 in human bladder cancer", CLIN. CANCER RES., vol. 12, no. 24, 15 December 2006 (2006-12-15), pages 7359-7368,
- DADUANG J. ET AL.: "Biomarker to distinguish hepatocellular carcinoma from cholangiocarcinoma by serum a disintegrin and metalloprotease 12", ARCH. MED. SCI., vol. 7, no. 6, 31 December 2011 (2011-12-31), pages 1013-1016,
- ROY R. ET AL.: "Potential of fluorescent metalloproteinase substrates for cancer detection", CLIN. BIOCHEM., vol. 44, no. 17-18, December 2011 (2011-12), pages 1434-1439, ISSN: 1873-2933
- CIREAP N. ET AL.: "Molecular profiling of ADAM12 and ADAM17 genes in human malignant melanoma", PATHOL. ONCOL. RES., vol. 19, no. 4, October 2013 (2013-10), pages 755-762,
- MINO N. ET AL.: "A disintegrin and metalloprotease 12 (ADAM12) is a prognostic factor in resected pathological stage I lung adenocarcinoma", J. SURG. ONCOL., vol. 100, no. 3, September 2009 (2009-09), pages 267-272,
- SHAO S. ET AL.: "ADAM-12 as a diagnostic marker for the proliferation, migration and invasion in patients with small cell lung cancer", PLOS ONE, vol. 9, no. 1, E85936, 21 January 2014 (2014-01-21), pages 1-10,

## Description

### FIELD OF THE INVENTION

The present invention relates to methods for the diagnosis and prognosis of cancers of epithelial origin by assessing levels of ADAM 12 in a biological sample obtained from a patient.

### BACKGROUND OF THE INVENTION

One of the most important factors in the survival of cancer is detection at an early stage. Clinical assays that detect the early events of cancer offer an opportunity to intervene and prevent cancer progression. With the development of gene profiling and proteomics there has been significant progress in the identification of molecular markers or "biomarkers" that can be used to diagnose and prognose specific cancers. For example, in the case of prostate cancer, the antigen PSA (for prostate specific antigen) can be detected in the blood and is indicative of the presence of prostate cancer. Thus, the blood of men at risk for prostate cancer can be quickly, easily, and safely screened for elevated PSA levels.

Even though there has been significant progress in the field of cancer detection, there still remains a need in the art for the identification of new biomarkers for a variety of cancers that can be easily used in clinical applications. For example, to date there are relatively few options available for the diagnosis of breast cancer using easily detectable biomarkers. Overexpression of EGFR, particularly coupled with down-regulation of the estrogen receptor, is a marker of poor prognosis in breast cancer patients. Other known markers of breast cancer include high levels of M2 pyruvate kinase (M2 PK) in blood (U.S. Patent No 6,358,683), high ZNF217 protein levels in blood (WO 98/02539), and differential expression of a newly identified protein in breast cancer, PDEBC, which is useful for diagnosis (U.S. patent application No. 20030124543). These biomarkers offer an alternative method of diagnosis, however, they are not widely used. Furthermore, despite the use of a number of histochemical, genetic, and immunological markers, clinicians still have a difficult time predicting which tumors will metastasize to other organs.

ADAM 12 (a disintegrin and metalloproteinase domain 12) known to mediate cell adhesion and spreading through syndecan and integrin interactions has recently been implicated as a marker for tumor aggressiveness and progression in liver cancer (Pabic et al. Hepatology (2003) vol. 37(5): 1056- 1066). However, detection of ADAM 12 levels in this study required biopsy and there was no disclosure regarding detection of ADAM 12 in serum or urine. Thus, ADAM 12 as disclosed by Pabic et al. apparently does not serve as an easily detectable biomarker.

The identification of biomarkers is particularly relevant to improving diagnosis, prognosis, and treatment of the disease. As such, there is need in the art to identify alternative biomarkers that can be quickly, easily, and safely detected. Such biomarkers may be used to diagnose, to stage, or to monitor the progression or treatment of a subject with breast cancer, in particular, an invasive, potentially metastatic stage of the disease.

WO 02/059377 describes methods for diagnosing breast cancer, and mentions ADAM 12 in Table 4, which is said to show genes upregulated in tumor tissue compared to normal breast tissue. There is no teaching of a method of diagnosing breast cancer by analysing ADAM 12 levels in a sample obtained non-invasively from the patient.

WO 97/40072 describes ADAM 12 proteins but does not teach a method for facilitating the diagnosis of a patient for a cancer of epithelial origin.

US 2002/081641 describes a non-invasive enzyme screen for tissue remodelling-associated conditions, including cancer of epithelial origin. The screen is based on the detection of matrix metalloproteinases. There is no teaching that ADAM 12 is upregulated in cancers of epithelial origin and that these can be used in the detection of epithelial cancers.

Hepatology, Vol. 37, No. 5, May 2003, pages 1056 to 1066, describes that ADAM 12 expression is increased in human liver cancers on the basis of tests of samples from livers with hepatocellular carcinomas and from livers with metastases from colorectal cancers. There is no teaching of a method of diagnosing cancer or a method for prognostic evaluation of a patient suspected of having cancer by analysing ADAM 12 levels in a sample obtained non-invasively from the patient.

### SUMMARY OF THE INVENTION

The present invention is based on the surprising discovery that ADAM 12 (both active and latent) protein is present in urine and that ADAM 12 expression and activity are up regulated in patients that have cancers of epithelial origin, such as breast cancer, colon cancer, prostate cancer, liver cancer, bladder cancer, pancreatic cancer, ovarian cancer, cervical cancer, lung cancer, and skin cancer, as well as others. Accordingly, the present invention is directed to methods for prognostic evaluation, facilitating diagnosis of cancers of epithelial origin and as a marker for therapeutic efficacy. In particular, the amount of ADAM 12 protein detected in biological samples, e.g. urine, correlate with disease status such that ADAM 12 levels can be used to predict the presence of, as well as the metastatic potential of cancer. Thus, measuring the level of ADAM 12 in biological samples (e.g. urine or blood) provides a quick, easy, and safe screen that can be used to both diagnose and prognose cancer of epithelial origin, e.g., breast cancer in a patient.

In one embodiment, a method for facilitating the diagnosis of cancer of epithelial origin in a patient is provided. The method comprises obtaining a biological sample from a patient and detecting the presence or absence of ADAM 12 (or a fragment thereof) in the biological sample, wherein the presence of ADAM 12 is indicative of the presence of cancer of epithelial origin.

In another embodiment, the method comprises measuring the level of ADAM 12 present in a test biological sample from a patient and comparing the observed level of ADAM 12 with the level of ADAM 12 present in a control sample of the same type. Higher levels of ADAM 12 in the test sample, as compared to the control sample, is indicative of cancer. Preferably the methods of the invention are used for early detection of cancers of epithelial origin, especially breast cancer. For example, a patient can be screened annually by a physician during their annual physicals.

The term "control sample" refers to a biological sample obtained from a "normal" or "healthy" individual(s) that is believed not to have cancer. Controls may be selected using methods that are well known in the art. Once a level has become well established for a control population, array results from test biological samples can be directly compared with the known levels.

The term "test sample" refers to a biological sample obtained from a patient being tested for a cancer of epithelial origin.

Biological samples, for example, can be obtained from blood, tissue (e.g. tumor or breast), serum, stool, urine, sputum, cerebrospinal fluid, nipple aspirates and supernatant from cell lysate. One preferred biological sample is urine.

In one aspect, the cancer of epithelial origin to be diagnosed is breast cancer, basal cell carcinoma, adenocarcinoma, gastrointestinal cancer, such as, for example, lip cancer, mouth cancer, esophageal cancer, small bowel cancer and stomach cancer, colon cancer, liver cancer, bladder cancer, pancreas cancer, ovary cancer, cervical cancer, lung cancer, and skin cancer, such as squamous cell and basal cell cancers, prostate cancer, renal cell carcinoma, and other known cancers that effect epithelial cells throughout the body.

In another embodiment, the present invention provides a method for prognostic evaluation of a patient suspected of having, or having, a cancer of epithelial origin. The method comprises the steps of i) measuring the level of ADAM 12 present in a test sample obtained from the patient, ii) comparing the level of ADAM 12 in the test sample to a level of ADAM 12 protein known to be present in biological samples of the same type, which are obtained from healthy patients that are believed not to have cancer (a control sample), and iii) evaluating the prognosis of the patient based on the comparison, where a level of ADAM 12 in the test sample that is at least 3-fold greater than the level in the control sample indicates an aggressive form of cancer (e.g. metastatic or invasive) and therefore a poor prognosis.

The present invention also contemplates the assessment of the level of ADAM 12 present in multiple test samples obtained from the same patient, where a progressive increase in the amount of ADAM 12 over time indicates an increased aggressiveness (e.g. metastatic potential) of the cancer tumor. As such, the levels of ADAM 12 serve as a predictor of disease status and stage.

The present invention further contemplates the assessment of ADAM 12 levels to monitor the therapeutic efficacy of a treatment regime designed to treat a patient having a cancer of epithelial origin.

In one preferred embodiment, the biological samples are urine samples. However, biological samples of blood, tissue, serum, stool, sputum, cerebrospinal fluid, nipple aspirates, and supernatant from cell lysate can also be used.

In one aspect of the invention, ADAM 12 levels present in a test biological sample are measured by contacting the test sample, or preparation thereof, with an antibody-based binding moiety that specifically binds to ADAM 12 protein, or to a portion thereof. The antibody-based binding moiety forms a complex with ADAM 12 that can be detected, thereby allowing the levels of ADAM 12 to be measured.

Antibody-based immunoassays are the preferred means for measuring levels of ADAM 12 protein. However, any means known to those skilled in art can be used to assess ADAM 12 levels. For example, in some embodiments ADAM 12 expression levels are assayed by measuring levels of ADAM 12 mRNA transcripts. Alternatively, ADAM 12 levels can be assessed by mass spectrometry, including SELDI mass spectrometry. ADAM 12 levels can also be assessed by a biological activity assay including, but not limited to, substrate gel electrophoresis.

The present disclosure also describes kits that comprise means for measuring ADAM 12 in a biological sample.

Other aspects of the invention are disclosed infra.

### BRIEF DESCRIPTION OF THE DRAWINGS

The accompanying drawings, which are incorporated in and constitute a part of this specification, illustrate embodiments of the invention and, together with the description, serve to explain the objects, advantages, and principles of the invention.
Figure 1 shows an immunoblot analysis of ADAM 12 in urine samples obtained from patients with breast cancer as compared to urine samples obtained from age and sex matched normal controls; patients without breast cancer.
Figure 2 shows an immunoblot analysis of ADAM 12 in urine samples obtained from patients with various stages of breast cancer as compared to the level of ADAM 12 in a urine samples obtained from normal healthy individuals. Lane 11 represents pure recombinant ADAM 12 including a 92 kDa latent form and a 68 kDa mature form of the protein.
Figure 3 shows a histogram of the densitometry of the immunoblots of all samples. The histogram indicates the increase in median levels of urinary ADAM 12 with advanced stages of breast cancer. The error bars denote standard errors (*, p, 0.01, analysis of variance).

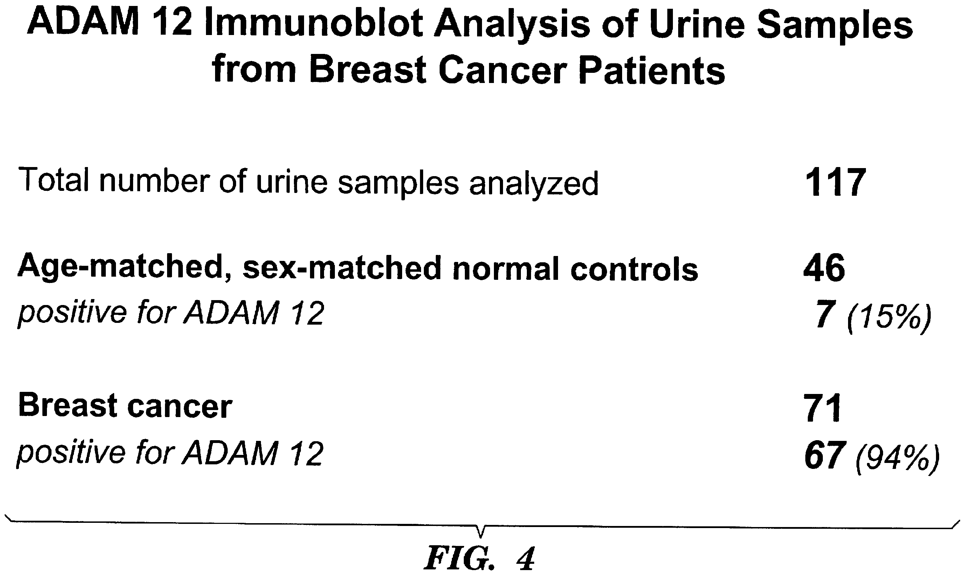

Figure 4 shows a chart that illustrates the correlation of the presence of ADAM 12 with breast cancer. 94% of the urine samples from cancer patients were positive for ADAM 12 in contrast to the normal urine samples in which ADAM 12 was detected with significantly lower frequency (15%).
Figure 5 shows the amino acid sequence for ADAM 12 (SEQ ID NO:1).
Figure 6 shows the percentage of patients with urinary ADAM 12 in breast cancer groups and controls. The mean densitometric score for control samples as threshold, and percent positive for each cancer group was calculated relative to the control group.
Figure 7 shows a theoretical curve based on logistic regression analysis illustrating the probability of having breast cancer (a opposed to normal) based on rising levels of ADAM 12. The probability curve is superimposed on the percentage of controls and breast cancer patients for specific intervals of ADAM 12 level (p< 0.00001). The x axis represents intervals of ADAM 12 level, and the y axis corresponds to the actual percentage of patients and controls in each interval.

### DETAILED DESCRIPTION OF THE INVENTION

We have discovered that the levels of ADAM 12 present in biological samples of patients correlate with the presence, or absence of, cancers of epithelial origin. In addition, it has been determined that high levels of ADAM 12 correlate with the aggressiveness and the metastatic potential of cancer tumors.

As used herein, "cancers of epithelial origin" refers to cancers that arise from epithelial cells which include, but are not limited to, breast cancer, basal cell carcinoma, adenocarcinoma, gastrointestinal cancer, lip cancer, mouth cancer, esophageal cancer, small bowel cancer and stomach cancer, colon cancer, liver cancer, bladder cancer, pancreas cancer, ovary cancer, cervical cancer, lung cancer, breast cancer and skin cancer, such as squamous cell and basal cell cancers, prostate cancer, renal cell carcinoma, and other known cancers that effect epithelial cells throughout the body.

The term "aggressive" or "invasive" with respect to cancer refers to the proclivity of a tumor for expanding beyond its boundaries into adjacent tissue (Darnell, J. (1990), Molecular Cell Biology, Third Ed., W. H. Freeman, NY). Invasive cancer can be contrasted with organ-confined cancer wherein the tumor is confined to a particular organ. The invasive property of a tumor is often accompanied by the elaboration of proteolytic enzymes, such as collagenases, that degrade matrix material and basement membrane material to enable the tumor to expand beyond the confines of the capsule, and beyond confines of the particular tissue in which that tumor is located.

The term "metastasis", as used herein, refers to the condition of spread of cancer from the organ of origin to additional distal sites in the patient. The process of tumor metastasis is a multistage event involving local invasion and destruction of intercellular matrix, intravasation into blood vessels, lymphatics or other channels of transport, survival in the circulation, extravasation out of the vessels in the secondary site and growth in the new location (Fidler, et al., Adv. Cancer Res. 28, 149-250 (1978), Liotta, et al., Cancer Treatment Res. 40, 223-238 (1988), Nicolson, Biochim. Biophy. Acta 948, 175-224 (1988) and Zetter, N. Eng. J. Med. 322, 605-612 (1990)). Increased malignant cell motility has been associated with enhanced metastatic potential in animal as well as human tumors (Hosaka, et al., Gann 69, 273-276 (1978) and Haemmerlin, et al., Int. J. Cancer 27, 603-610 (1981)).

As used herein, a "biological sample" refers to a sample of biological material obtained from a patient, preferably a human patient, including a tissue, a tissue sample, a cell sample (e. g., a tissue biopsy, such as, an aspiration biopsy, a brush biopsy, a surface biopsy, a needle biopsy, a punch biopsy, an excision biopsy, an open biopsy, an incision biopsy or an endoscopic biopsy), and a tumor sample. Biological samples can also be biological fluid samples. In one preferred embodiment the biological sample is urine. However, blood, serum, saliva, cerebrospinal fluid, nipple aspirates, and supernatant from cell lysate can also be used.

The present invention also encompasses the use of isolates of a biological sample in the methods of the invention. As used herein, an "isolate" of a biological sample (e. g., an isolate of a tissue or tumor sample) refers to a material or composition (e. g., a biological material or composition) which has been separated, derived, extracted, purified or isolated from the sample and preferably is substantially free of undesirable compositions and/or impurities or contaminants associated with the biological sample.

In a preferred embodiment, the biological sample is treated as to prevent degradation of ADAM 12 protein, or ADAM 12 mRNA. Methods for inhibiting or preventing degradation include, but are not limited to, treatment of the biological sample with protease or RNAase inhibitors, freezing the biological sample, or placing the biological sample on ice. Preferably, prior to analysis, the biological samples or isolates are constantly kept under conditions as to prevent degradation of ADAM 12 protein, or ADAM 12 RNA.

As used herein, a "tissue sample" refers to a portion, piece, part, segment, or fraction of a tissue which is obtained or removed from an intact tissue of a subject, preferably a human subject. One preferred tissue sample is mammary tissue.

As used herein, a "tumor sample" refers to a portion, piece, part, segment, or fraction of a tumor, for example, a tumor which is obtained or removed from a subject (e. g., removed or extracted from a tissue of a subject), preferably a human subject.

As used herein, a "primary tumor" is a tumor appearing at a first site within the subject and can be distinguished from a "metastatic tumor" which appears in the body of the subject at a remote site from the primary tumor.

As used herein, "LCIS" refers to lobular carcinoma in situ. LCIS is also called lobular neoplasia and is sometimes classified as a type of noninvasive breast cancer. It does not penetrate through the wall of the lobules. Although it does not itself usually become an invasive cancer, women with this condition have a higher risk of developing an invasive breast cancer in the same or opposite breast.

As used herein, "DCIS" refers to ductal carcinoma in situ. Ductal carcinoma in situ is the most common type of noninvasive breast cancer. In DCIS, the malignant cells have not metastasized through the walls of the ducts into the fatty tissue of the breast. Comedocarcinoma is a type of DCIS that is more likely than other types of DCIS to come back in the same area after lumpectomy, and is more closely linked to eventual development of invasive ductal carcinoma than other forms of DCIS.

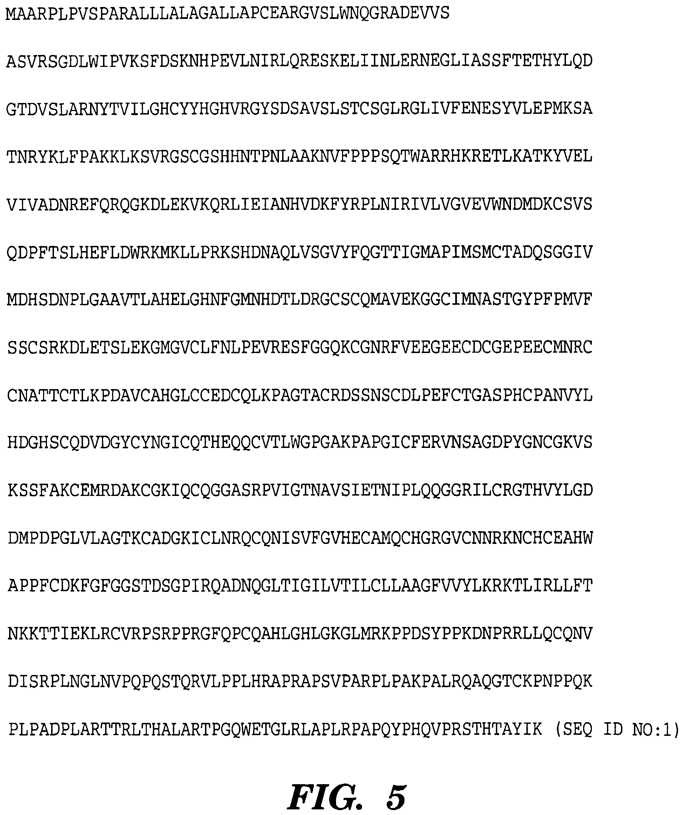

As used herein, "ADAM 12" refers to the ADAM 12 protein of Genebank accession NM_003474 (Homosapiens) (SEQ ID NO:1) (Fig.5). The term also encompasses species variants, homologues, allelic forms, mutant forms, and equivalents thereof.

The present invention is directed to methods for facilitating diagnosis of cancer of epithelial origin in a patient. In one embodiment, the method comprises obtaining a biological sample from a patient and detecting the presence or absence of ADAM 12 (or a fragment thereof) in the biological sample, wherein the presence of ADAM 12 is indicative of the presence of cancer of epithelial origin.

In another embodiment, the methods involve measuring levels of ADAM 12 in a test sample obtained from a patient, suspected of having cancer, and comparing the observed levels to levels of ADAM 12 found in a control sample, for example a sample obtained from an individual patient or population of individuals that are believed not to have cancer. Levels of ADAM 12 higher than levels that are observed in the normal control indicate the presence of cancer. The levels of ADAM 12 can be represented by arbitrary units, for example as units obtained from a densitometer, luminometer, or an Elisa plate reader.

As used herein, "a higher level of ADAM 12 in the test sample as compared to the level in the control sample" refers to an amount of ADAM 12 that is greater than an amount of ADAM 12 present in a control sample. The term "higher level" refers to a level that is statistically significant or significantly above levels found in the control sample. Preferably, the "higher level" is at least 2 fold greater. A level of ADAM 12 protein that indicates an aggressive form of cancer refers to amounts of ADAM 12 that are at least 3 fold greater than the amounts of ADAM 12 present in control samples, preferably 5 fold to 6 fold greater.

The term "statistically significant" or "significantly" refers to statistical significance and generally means a two standard deviation (2SD) above normal, or higher, concentration of the marker.

For purposes of comparison, the test sample and control sample are of the same type, that is, obtained from the same biological source. The control sample can also be a standard sample that contains the same concentration of ADAM 12 that is normally found in a biological sample of the same type and that is obtained from a healthy individual. For example, there can be a standard normal control sample for the amounts of ADAM 12 normally found in biological samples such as urine, blood, cerebral spinal fluid, or tissue.

In one embodiment, the methods described herein are used to detect breast cancer. Any type of breast cancer can be detected. For example, adenocarcinoma, ductal carcinoma in situ (DCIS), infiltrating (or invasive) ductal carcinoma (IDC), infiltrating (or invasive) lobular carcinoma (ILC), inflammatory breast cancer, in situ, lobular carcinoma in situ (LCIS), medullary carcinoma, mucinous carcinoma, Paget's disease of the nipple, Phyllodes tumor, and tubular carcinoma can be detected. In addition, a breast cancer at any stage of progression can be detected, such as primary, metastatic, and recurrent breast cancer. Information regarding numerous types of breast cancer can be found, e.g., from the American Cancer Society, or from, e.g., Wilson et al. (1991) Harrison's Principles of Internal Medicine, 12th Edition, McGraw-Hill, Inc.

In one aspect of the invention, a secondary diagnostic step can be performed. For example, if a level of ADAM 12 is found to indicate the presence of cancer, then an additional method of detecting the cancer can be performed to confirm the presence of the cancer. Any of a variety of additional diagnostic steps can be used, such as mammography (breast cancer), ultrasound, PET scanning, MRI, or any other imaging techniques, biopsy, clinical examination, ductogram, or any other method.

The present invention further provides for methods of prognostic evaluation of a patient suspected of having, or having, cancer of epithelial origin. The method comprises measuring the level of ADAM 12 present in a test biological sample obtained from a patient and comparing the observed level with a range of ADAM 12 levels normally found in biological samples (of the same type) of healthy individuals. A high level for example, is indicative of a greater potential for metastatic activity and corresponds to a poor prognosis, while lower levels indicate that the tumor is less aggressive and correspond to a better prognosis.

Additionally, disease progression can be assessed by following ADAM 12 levels in an individual patient. For example, changes in the patients condition can be monitored by comparing changes in ADAM 12 expression levels in the patient over time. Progressive increases in ADAM 12 levels is indicative of increased potential for tumor invasion and metastasis.

The prognostic methods of the invention also are useful for determining a proper course of treatment for a patient having cancer. A course of treatment refers to the therapeutic measures taken for a patient after diagnosis or after treatment for cancer. For example, a determination of the likelihood for cancer recurrence, spread, or patient survival, can assist in determining whether a more conservative or more radical approach to therapy should be taken, or whether treatment modalities should be combined. For example, when cancer recurrence is likely, it can be advantageous to precede or follow surgical treatment with chemotherapy, radiation, immunotherapy, biological modifier therapy, gene therapy, vaccines, and the like, or adjust the span of time during which the patient is treated.

### Measuring levels ofADAM 12

The levels of ADAM 12, as described herein, can be measured by any means known to those skilled in the art. In the present invention, it is generally preferred to use antibodies, or antibody equivalents, to detect levels of ADAM 12 protein in biological samples. However, other methods for detection of ADAM 12 expression can also be used, such as measuring ADAM 12 expression by analysis of mRNA transcripts. Measuring ADAM 12 mRNA may be preferred, for example when the biological sample is a tumor, or tissue sample.

Methods for assessing levels of mRNA are well known to those skilled in the art. For example, detection of RNA transcripts may be achieved by Northern blotting, wherein a preparation of RNA is run on a denaturing agarose gel, and transferred to a suitable support, such as activated cellulose, nitrocellulose or glass or nylon membranes. Labeled (e.g., radiolabeled) cDNA or RNA is then hybridized to the preparation, washed and analyzed by methods such as autoradiography.

Detection of RNA transcripts can further be accomplished using known amplification methods. For example, it is within the scope of the present invention to reverse transcribe mRNA into cDNA followed by polymerase chain reaction (RT-PCR); or, to use a single enzyme for both steps as described in U.S. Pat. No. 5,322,770, or reverse transcribe mRNA into cDNA followed by symmetric gap lipase chain reaction (RT-AGLCR) as described by R. L. Marshall, et al., PCR Methods and Applications 4: 80-84 (1994). One suitable method for detecting ADAM 12 mRNA transcripts is described in reference Pabic et. al. Hepatology, 37(5): 1056-1066, 2003.

Other known amplification methods which can be utilized herein include but are not limited to the so-called "NASBA" or "3SR" technique described in PNAS USA 87: 1874-1878 (1990) and also described in Nature 350 (No. 6313): 91-92 (1991); Q-beta amplification as described in published European Patent Application (EPA) No. 4544610; strand displacement amplification (as described in G. T. Walker et al., Clin. Chem. 42: 9-13 (1996) and European Patent Application No. 684315; and target mediated amplification, as described by PCT Publication WO 9322461.

In situ hybridization visualization may also be employed, wherein a radioactively labeled antisense RNA probe is hybridized with a thin section of a biopsy sample, washed, cleaved with RNase and exposed to a sensitive emulsion for autoradiography. The samples may be stained with haematoxylin to demonstrate the histological composition of the sample, and dark field imaging with a suitable light filter shows the developed emulsion. Non-radioactive labels such as digoxigenin may also be used.

Alternatively, mRNA expression can be detected on a DNA array, chip or a microarray. Oligonucleotides corresponding to ADAM 12 are immobilized on a chip which is then hybridized with labeled nucleic acids of a test sample obtained from a patient. Positive hybridization signal is obtained with the sample containing ADAM 12 transcripts. Methods of preparing DNA arrays and their use are well known in the art. (See, for example U.S. Patent Nos: 6,618,6796; 6,379,897; 6,664,377; 6,451,536; 548,257; U.S. 20030157485 and Schena et al. 1995 Science 20:467-470; Gerhold et al. 1999 Trends in Biochem. Sci. 24, 168-173; and Lennon et al. 2000 Drug discovery Today 5: 59-65). Serial Analysis of Gene Expression (SAGE) can also be performed (See for example U.S. Patent Application 20030215858).

To monitor mRNA levels, for example, mRNA is extracted from the biological sample to be tested, reverse transcribed, and fluorescent-labeled cDNA probes are generated. The microarrays capable of hybridizing to ADAM 12 cDNA are then probed with the labeled cDNA probes, the slides scanned and fluorescence intensity measured. This intensity correlates with the hybridization intensity and expression levels.

ADAM 12 protein levels, or ADAM 12 activity, can also be measured, in particular, when the biological sample is a fluid sample such as blood or urine. In one embodiment, levels of ADAM 12 protein are measured by contacting the biological sample with an antibody-based binding moiety that specifically binds to ADAM 12, or to a fragment of ADAM 12. Formation of the antibody-ADAM 12 complex is then detected as a measure of ADAM 12 levels.

The term "antibody-based binding moiety" or "antibody" includes immunoglobulin molecules and immunologically active determinants of immunoglobulin molecules, e.g., molecules that contain an antigen binding site which specifically binds (immunoreacts with) to ADAM 12. The term "antibody-based binding moiety" is intended to include whole antibodies, e.g., of any isotype (IgG, IgA, IgM, IgE, etc), and includes fragments thereof which are also specifically reactive with ADAM 12 protein. Antibodies can be fragmented using conventional techniques. Thus, the term includes segments of proteolytically-cleaved or recombinantly-prepared portions of an antibody molecule that are capable of selectively reacting with a certain protein. Non limiting examples of such proteolytic and/or recombinant fragments include Fab, F(ab')2, Fab' , Fv, dAbs and single chain antibodies (scFv) containing a VL and VH domain joined by a peptide linker. The scFv's may be covalently or non-covalently linked to form antibodies having two or more binding sites. Thus, "antibody-base binding moiety" includes polyclonal, monoclonal, or other purified preparations of antibodies and recombinant antibodies. The term "antibody-base binding moiety" is further intended to include humanized antibodies, bispecific antibodies, and chimeric molecules having at least one antigen binding determinant derived from an antibody molecule. In a preferred embodiment, the antibody-based binding moiety detectably labeled.

"Labeled antibody", as used herein, includes antibodies that are labeled by a detectable means and include, but are not limited to, antibodies that are enzymatically, radioactively, fluorescently, and chemiluminescently labeled. Antibodies can also be labeled with a detectable tag, such as c-Myc, HA, VSV-G, HSV, FLAG, V5, or HIS.

In the diagnostic and prognostic methods of the invention that use antibody based binding moieties for the detection of ADAM 12, the level of ADAM 12 present in the biological samples correlate to the intensity of the signal emitted from the detectably labeled antibody.

In one preferred embodiment, the antibody-based binding moiety is detectably labeled by linking the antibody to an enzyme. The enzyme, in turn, when exposed to it's substrate, will react with the substrate in such a manner as to produce a chemical moiety which can be detected, for example, by spectrophotometric, fluorometric or by visual means. Enzymes which can be used to detectably label the antibodies of the present invention include, but are not limited to, malate dehydrogenase, staphylococcal nuclease, delta-V-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholinesterase. Chemiluminescence is another method that can be used to detect an antibody-based binding moiety.

Detection may also be accomplished using any of a variety of other immunoassays. For example, by radioactively labeling an antibody, it is possible to detect the antibody through the use of radioimmune assays. The radioactive isotope can be detected by such means as the use of a gamma counter or a scintillation counter or by audoradiography. Isotopes which are particularly useful for the purpose of the present invention are ³H, ¹³¹I, ³⁵S, ¹⁴C, and preferably ¹²⁵I.

It is also possible to label an antibody with a fluorescent compound. When the fluorescently labeled antibody is exposed to light of the proper wave length, its presence can then be detected due to fluorescence. Among the most commonly used fluorescent labeling compounds are CYE dyes, fluorescein isothiocyanate, rhodamine, phycoerytherin, phycocyanin, allophycocyanin, o-phthaldehyde and fluorescamine.

An antibody can also be detectably labeled using fluorescence emitting metals such as ¹⁵²Eu, or others of the lanthanide series. These metals can be attached to the antibody using such metal chelating groups as diethylenetriaminepentaacetic acid (DTPA) or ethylenediaminetetraacetic acid (EDTA).

An antibody also can be detectably labeled by coupling it to a chemiluminescent compound. The presence of the chemiluminescent-antibody is then determined by detecting the presence of luminescence that arises during the course of a chemical reaction. Examples of particularly useful chemiluminescent labeling compounds are luminol, luciferin, isoluminol, theromatic acridinium ester, imidazole, acridinium salt and oxalate ester.

As mentioned above, levels of ADAM 12 protein can be detected by immunoassays, such as enzyme linked immunoabsorbant assay (ELISA), radioimmunoassay (RIA), Immunoradiometric assay (IRMA), Western blotting, or immunohistochemistry, each of which are described in more detail below. Immunoassays such as ELISA or RIA, which can be extremely rapid, are more generally preferred. Antibody arrays or protein chips can also be employed, see for example U.S. Patent Application Nos: 20030013208A1; 20020155493A1; 20030017515 and U.S. Patent Nos: 6,329,209; 6,365,418.

### Immunoassays

"Radioimmunoassay" is a technique for detecting and measuring the concentration of an antigen using a labeled (e.g.. radioactively labeled) form of the antigen. Examples of radioactive labels for antigens include ³H, .¹⁴C, and ¹²⁵I. The concentration of antigen ADAM 12 in a biological sample is measured by having the antigen in the biological sample compete with the labeled (e.g. radioactively) antigen for binding to an antibody to the antigen. To ensure competitive binding between the labeled antigen and the unlabeled antigen, the labeled antigen is present in a concentration sufficient to saturate the binding sites of the antibody. The higher the concentration of antigen in the sample, the lower the concentration of labeled antigen that will bind to the antibody.

In a radioimmunoassay, to determine the concentration of labeled antigen bound to antibody, the antigen-antibody complex must be separated from the free antigen. One method for separating the antigen-antibody complex from the free antigen is by precipitating the antigen-antibody complex with an anti-isotype antiserum. Another method for separating the antigen-antibody complex from the free antigen is by precipitating the antigen-antibody complex with formalin-killed S. aureus. Yet another method for separating the antigen-antibody complex from the free antigen is by performing a "solid-phase radioimmunoassay" where the antibody is linked (e.g., covalently) to Sepharose beads, polystyrene wells, polyvinylchloride wells, or microtiter wells. By comparing the concentration of labeled antigen bound to antibody to a standard curve based on samples having a known concentration of antigen, the concentration of antigen in the biological sample can be determined.

A "Immunoradiometric assay" (IRMA) is an immunoassay in which the antibody reagent is radioactively labeled. An IRMA requires the production of a multivalent antigen conjugate, by techniques such as conjugation to a protein e.g., rabbit serum albumin (RSA). The multivalent antigen conjugate must have at least 2 antigen residues per molecule and the antigen residues must be of sufficient distance apart to allow binding by at least two antibodies to the antigen. For example, in an IRMA the multivalent antigen conjugate can be attached to a solid surface such as a plastic sphere. Unlabeled "sample" antigen and antibody to antigen which is radioactively labeled are added to a test tube containing the multivalent antigen conjugate coated sphere. The antigen in the sample competes with the multivalent antigen conjugate for antigen antibody binding sites. After an appropriate incubation period, the unbound reactants are removed by washing and the amount of radioactivity on the solid phase is determined. The amount of bound radioactive antibody is inversely proportional to the concentration of antigen in the sample.

The most common enzyme immunoassay is the "Enzyme-Linked Immunosorbent Assay (ELISA)." ELISA is a technique for detecting and measuring the concentration of an antigen using a labeled (e.g. enzyme linked) form of the antibody. There are different forms of ELISA, which are well known to those skilled in the art. The standard techniques known in the art for ELISA are described in "Methods in Immunodiagnosis", 2nd Edition, Rose and Bigazzi, eds. John Wiley & Sons, 1980; Campbell et al., "Methods and Immunology", W. A. Benjamin, Inc., 1964; and Oellerich, M. 1984, J. Clin. Chem. Clin. Biochem., 22:895-904.

In a "sandwich ELISA", an antibody (e.g. anti-ADAM 12) is linked to a solid phase (i.e. a microtiter plate) and exposed to a biological sample containing antigen (e.g. ADAM 12). The solid phase is then washed to remove unbound antigen. A labeled antibody (e.g. enzyme linked) is then bound to the bound-antigen (if present) forming an antibody-antigen-antibody sandwich. Examples of enzymes that can be linked to the antibody are alkaline phosphatase, horseradish peroxidase, luciferase, urease, and B-galactosidase. The enzyme linked antibody reacts with a substrate to generate a colored reaction product that can be measured.

In a "competitive ELISA", antibody is incubated with a sample containing antigen (i.e. ADAM 12). The antigen-antibody mixture is then contacted with a solid phase (e.g. a microtiter plate) that is coated with antigen (i.e., ADAM 12). The more antigen present in the sample, the less free antibody that will be available to bind to the solid phase. A labeled (e.g., enzyme linked) secondary antibody is then added to the solid phase to determine the amount of primary antibody bound to the solid phase.

In a "immunohistochemistry assay" a section of tissue is tested for specific proteins by exposing the tissue to antibodies that are specific for the protein that is being assayed. The antibodies are then visualized by any of a number of methods to determine the presence and amount of the protein present. Examples of methods used to visualize antibodies are, for example, through enzymes linked to the antibodies (e.g., luciferase, alkaline phosphatase, horseradish peroxidase, or .beta.-galactosidase), or chemical methods (e.g., DAB/Substrate chromagen).

Other techniques may be used to detect ADAM 12, according to a practitioner's preference, based upon the present disclosure. One such technique is Western blotting (Towbin et at., Proc. Nat. Acad. Sci. 76:4350 (1979)), wherein a suitably treated sample is run on an SDS-PAGE gel before being transferred to a solid support, such as a nitrocellulose filter. Detectably labeled anti-ADAM 12 antibodies can then be used to assess ADAM 12 levels, where the intensity of the signal from the detectable label corresponds to the amount of ADAM 12 present. Levels can be quantitated, for example by densitometry.

### Mass Spectometry

In addition, ADAM 12 may be detected using Mass Spectrometry such as MALDI/TOF (time-of-flight), SELDI/TOF, liquid chromatography-mass spectrometry (LC-MS), gas chromatography-mass spectrometry (GC-MS), high performance liquid chromatography-mass spectrometry (HPLC-MS), capillary electrophoresis-mass spectrometry, nuclear magnetic resonance spectrometry, or tandem mass spectrometry (e.g., MS/MS, MS/MS/MS, ESI-MS/MS, etc.). See for example, U.S. Patent Application Nos: 20030199001, 20030134304, 20030077616.

Mass spectrometry methods are well known in the art and have been used to quantify and/or identify biomolecules, such as proteins (see, e.g., Li et al. (2000) Tibtech 18:151-160; Rowley et al. (2000) Methods 20: 383-397; and Kuster and Mann (1998) Curr. Opin. Structural Biol. 8: 393-400). Further, mass spectrometric techniques have been developed that permit at least partial de novo sequencing of isolated proteins. Chait et al., Science 262:89-92 (1993); Keough et al., Proc. Natl. Acad. Sci. USA. 96:7131-6 (1999); reviewed in Bergman, EXS 88:133-44 (2000).

In certain embodiments, a gas phase ion spectrophotometer is used. In other embodiments, laser-desorption/ionization mass spectrometry is used to analyze the sample. Modern laser desorption/ionization mass spectrometry ("LDI-MS") can be practiced in two main variations: matrix assisted laser desorption/ionization ("MALDI") mass spectrometry and surface-enhanced laser desorption/ionization ("SELDI"). In MALDI, the analyte is mixed with a solution containing a matrix, and a drop of the liquid is placed on the surface of a substrate. The matrix solution then co-crystallizes with the biological molecules. The substrate is inserted into the mass spectrometer. Laser energy is directed to the substrate surface where it desorbs and ionizes the biological molecules without significantly fragmenting them. However, MALDI has limitations as an analytical tool. It does not provide means for fractionating the sample, and the matrix material can interfere with detection, especially for low molecular weight analytes. See, e.g., U.S. Pat. No. 5,118,937 (Hillenkamp et al.), and U.S. Pat. No. 5,045,694 (Beavis & Chait).

In SELDI, the substrate surface is modified so that it is an active participant in the desorption process. In one variant, the surface is derivatized with adsorbent and/or capture reagents that selectively bind the protein of interest. In another variant, the surface is derivatized with energy absorbing molecules that are not desorbed when struck with the laser. In another variant, the surface is derivatized with molecules that bind the protein of interest and that contain a photolytic bond that is broken upon application of the laser. In each of these methods, the derivatizing agent generally is localized to a specific location on the substrate surface where the sample is applied. See, e.g., U.S. Pat. No. 5,719,060 and WO 98/59361. The two methods can be combined by, for example, using a SELDI affinity surface to capture an analyte and adding matrix-containing liquid to the captured analyte to provide the energy absorbing material.

For additional information regarding mass spectrometers, see, e.g., Principles of Instrumental Analysis, 3rd edition., Skoog, Saunders College Publishing, Philadelphia, 1985; and Kirk-Othmer Encyclopedia of Chemical Technology, 4.sup.th ed. Vol. 15 (John Wiley & Sons, New York 1995), pp. 1071-1094.

Detection of the presence of a marker or other substances will typically involve detection of signal intensity. This, in turn, can reflect the quantity and character of a polypeptide bound to the substrate. For example, in certain embodiments, the signal strength of peak values from spectra of a first sample and a second sample can be compared (e.g., visually, by computer analysis etc.), to determine the relative amounts of particular biomolecules. Software programs such as the Biomarker Wizard program (Ciphergen Biosystems, Inc., Fremont, Calif.) can be used to aid in analyzing mass spectra. The mass spectrometers and their techniques are well known to those of skill in the art.

Any person skilled in the art understands, any of the components of a mass spectrometer (e.g., desorption source, mass analyzer, detect, etc.) and varied sample preparations can be combined with other suitable components or preparations described herein, or to those known in the art. For example, in some embodiments a control sample may contain heavy atoms (e.g. ¹³C) thereby permitting the test sample to mixed with the known control sample in the same mass spectrometry run.

In one preferred embodiment, a laser desorption time-of-flight (TOF) mass spectrometer is used. In laser desorption mass spectrometry, a substrate with a bound marker is introduced into an inlet system. The marker is desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of molecules of specific mass to charge ratio.

In some embodiments the relative amounts of one or more biomolecules present in a first or second sample is determined, in part, by executing an algorithm with a programmable digital computer. The algorithm identifies at least one peak value in the first mass spectrum and the second mass spectrum. The algorithm then compares the signal strength of the peak value of the first mass spectrum to the signal strength of the peak value of the second mass spectrum of the mass spectrum. The relative signal strengths are an indication of the amount of the biomolecule that is present in the first and second samples. A standard containing a known amount of a biomolecule can be analyzed as the second sample to provide better quantify the amount of the biomolecule present in the first sample. In certain embodiments, the identity of the biomolecules in the first and second sample can also be determined.

In one preferred embodiment, ADAM 12 levels are measured by MALDI-TOF mass spectrometry.

### Other Assays

ADAM 12 levels can also be measured by using other biological assays, for example that measure activity, including but not limited to, zymography. Zymography is an assay well known to those skilled in the art and described in Heusen et al., Anal. Biochem., (1980) 102:196-202; Wilson et al., Journal of Urology, (1993) 149:653-658; Hernon et al., J. Biol. Chem. (1986) 261: 2814-2828, Braunhut et al., J. Biol. Chem. (1994) 269: 13472-13479; and Moses et al., Cancer Research 58, 1395-1399, April 1, 1998.

### ADAM 12 Antibodies

The antibodies for use in the present invention can be obtained from a commercial source. Alternatively, antibodies can be raised against ADAM 12 polypeptide, or a portion of ADAM 12 polypeptide. Methods for the production of ADAM 12 antibodies are disclosed in PCT publication WO 97/40072 or U.S. Application. No. 2002/0182702.

Antibodies for use in the present invention can be produced using standard methods to produce antibodies, for example, by monoclonal antibody production (Campbell, A.M., Monoclonal Antibodies Technology: Laboratory Techniques in Biochemistry and Molecular Biology, Elsevier Science Publishers, Amsterdam, the Netherlands (1984); St. Groth et al., J. Immunology, (1990) 35: 1-21; and Kozbor et al., Immunology Today (1983) 4:72). Antibodies can also be readily obtained by using antigenic portions of the protein to screen an antibody library, such as a phage display library by methods well known in the art. For example, U.S. patent 5,702,892 (U.S.A. Health & Human Services) and WO 01/18058 (Novopharm Biotech Inc.) disclose bacteriophage display libraries and selection methods for producing antibody binding domain fragments.

### ADAM 12 Detection Kit

The present disclosure also describes commercial kits for the detection and prognostic evaluation of a cancer of epithelial origin. The kit can be in any configuration well known to those of ordinary skill in the art and is useful for performing one or more of the methods described herein for the detection of ADAM 12. The kits are convenient in that they supply many if not all of the essential reagents for conducting an assay for the detection of ADAM 12 in a biological sample. In addition, the assay is preferably performed simultaneously with a standard or multiple standards that are included in the kit, such as a predetermined amount of ADAM 12 protein or nucleic acid, so that the results of the test can be quantitated or validated.

The kits include a means for detecting ADAM 12 levels such as antibodies, or antibody fragments, which selectively bind to ADAM 12 protein, or a set of DNA oligonucleotide primers that allows synthesis of cDNA encoding the protein, or for example, a DNA probe that detects expression of ADAM 12 mRNA. The diagnostic assay kit is preferentially formulated in a standard two-antibody binding format in which one ADAM 12-specific antibody captures ADAM 12 in a patient sample and another ADAM-specific antibody is used to detect captured ADAM 12. For example, the capture antibody is immobilized on a solid phase, e.g., an assay plate, an assay well, a nitrocellulose membrane, a bead, a dipstick, or a component of an elution column. The second antibody, i.e., the detection antibody, is typically tagged with a detectable label such as a calorimetric agent or radioisotope.

In one preferred embodiment, the kit comprises a means for detecting levels of ADAM 12 in a sample of urine. In a specific embodiment, the kit comprises a "dipstick" with anti-ADAM 12 antibodies or fragments, immobilized thereon, which specifically bind ADAM 12 protein. Specifically bound ADAM 12 protein can then be detected using, for example, a second antibody that is detectably labeled with a calorimetric agent or radioisotope.

In other embodiments, the assay kits may employ (but are not limited to) the following techniques: competitive and non-competitive assays, radioimmunoassay (RIA), bioluminescence and chemiluminescence assays, fluorometric assays, sandwich assays, immunoradiometric assays, dot blots, enzyme linked assays including ELISA, microtiter plates, and immunocytochemistry. For each kit the range, sensitivity, precision, reliability, specificity and reproducibility of the assay are established by means well known to those skilled in the art.

The above described assay kits would further provide instructions for use.

The present invention is further illustrated by the following Examples.

These Examples are provided to illustrate the invention without limitation thereof.

### EXAMPLE 1 Correlation of Urinary ADAM 12 with disease status and stage in breast cancer

ADAMs (a disintegrin and metalloprotease) are a recently discovered family of integral membrane and secreted glycoproteins that are related to snake venom metalloproteases (SNMPs) and matrix metalloproteases (MMPs). Members of this family display diverse roles in cell-surface remodeling, ectodomain shedding, regulation of growth factor availability and in mediating cell-cell and cell-matrix interactions in both normal development and pathological states such as Alzheimer's disease, rheumatoid arthritis and cancer. Human ADAM 12 is expressed as two alternatively spliced forms, a membrane-anchored long form (ADAM 12-L), and a shorter secreted form (ADAM 12-S). Increased levels of ADAM 12 mRNA and protein have previously been reported in breast, colon, lung and hepatocellular carcinoma tissue. We have previously reported (Cancer Research, Vol. 58, No. 7, 1 April 1998, pages 1395-1399) that matrix metalloproteases (MMPs) can be detected in urine of patients with a variety of different cancers and that their presence is an independent predictor of disease status. However, other than MMPs, the presence of other proteases as disease markers in urine has not yet been thoroughly explored. The goal of the present study was to identify other proteases present in urine of cancer patients and to determine whether their presence might be relevant to disease status. Using a combination of chromatographic steps and MALDI-TOF mass spectrometry, we report the detection of ADAM 12 in the urine of breast cancer patients.

### MATERIALS AND METHODS

### Identification of urinary ADAM 12.

Initial identification of ADAM 12 was made using 50 ml of urine (total protein ~5 mg). Urine was concentrated by ultrafiltration using Amicon membrane XM-50 (Millipore Corporation, Bedford, MA). The sample was diluted with 2 vol of 50 mM Tris, pH 7.5 (buffer A) and applied to a Q Sepharose column. After washing, a gradient of 0 - 400 mM NaCl in buffer A was applied. Fractions containing gelatinase activity were pooled, the NaCl concentration adjusted to 200 mM and incubated with Gelatin Sepharose (Amersham Biosciences, Piscataway, NJ) for 16 h at 4 °C with constant shaking. After a wash step, elution was performed using a stepwise gradient of 5, 10, and 20% DMSO respectively in buffer A containing 200 mM NaCl. Eluted fractions were concentrated and separated via SDS-PAGE under non-reducing conditions and stained using Sterling Rapid Silver Stain Kit (National Diagnostics, Atlanta, GA) according to the manufacturer's instructions. Several distinct bands of ~52, 80, 120 and 140 kDa were detected. Protein bands were excised from the gel, subjected to tryptic digest and analyzed by MALDI time-of-flight (TOFMS) mass spectrometry (Perceptive STR, Applied Biosystems, Framingham, MA) to determine the molecular weights of the proteins and for peptide mapping of the tryptic digests using a 337-nm wavelength laser for desorption and the reflectron mode of analysis. Using the MSFit (Baker, P.R. and Clauser, K.R.) search program the peptide maps generated were searched against a FASTA database of public domain proteins constructed of protein entries in the non-redundant database held by the NCBI and Swiss-Prot. Peptide matches identified by MSFit were filtered according to their MOWSE score, percentage of masses matched, molecular weight and number of observations of peptides and proteins. For conservative identification of proteins at least two peptides with good scores that matched a single protein are required.

### Expression and purification of ADAM 12-S.

The full-length human ADAM 12-S gene was cloned into plasmid (p1151) and transfection of *Cos-7* cells (ATCC, CRL 1651) was conducted as previously described (5). Expressed recombinant ADAM 12-S secreted into the conditioned medium, was purified as previously described with minor modifications. Briefly, conditioned media from transfected *Cos*-7 cells was subjected to successive steps of SP Sepharose and ConA Sepharose chromatography as described (6). ADAM 12-S containing fractions from the ConA Sepharose column were pooled and after buffer exchange applied to a Vivapure H Mini Q column (VivaScience, Carlsbad, CA) pre-equilibrated with buffer A containing 0.05% CHAPS. Pure ADAM 12-S was detected in the flow-through fraction as determined by SDS-PAGE and immunoblot analysis.

### Substrate specificity studies.

Gelatin, Type IV collagen, fibronectin and casein-degrading activity of ADAM 12-S was measured as previously described (14). ADAM 12-S (1 µM) was mixed with non-reducing sample buffer and separated on a 10% polyacrylamide gel containing either 0.1 % gelatin, 0.1 % Type IV collagen or 0.02% fibronectin and a 12% polyacrylamide gel containing 0.1% casein. After electrophoresis, the gel was washed with 2.5% Triton X-100 for 30 min. Substrate digestion was conducted by incubating the gel in 50 mM Tris, pH 7.6, containing 5 mM CaCl₂, 1 µM ZnCl₂ and 0.02% NaN₃ at 37 °C for 18 h. Gels were stained with 0.1% Coomassie and bands of enzyme activity were detected as zones of clearance on a background of uniform blue staining. To determine whether ADAM 12 possessed Type I collagen-degrading activity, pure enzyme (1 µM) was analyzed using a radiometric collagenase assay as described previously (16). For inhibition assays, ADAM 12 (160 nM) was incubated with 30 µg gelatin in assay buffer (50 mM Tris, pH 7.5) in the presence or absence of various inhibitors 5 mM 1, 10-phenanthroline, 5 mM EDTA, 1 mM Marimastat or two different concentrations (100 and 500 nM) of either TIMP-1, TIMP-2, TIMP-3 or TIMP-4 in a final volume of 30 µl at 37 °C for 18 h. Reactions were stopped with the addition of non-reducing sample buffer and the samples resolved by SDS-PAGE analysis followed by Coomassie staining.

### Urine sample collection and processing.

Urine collection was performed as previously described (14). Samples were collected in sterile containers and immediately frozen at -20 °C. Urine was collected according to the institutional bioethical guidelines pertaining to discarded clinical material. Prior to analysis, urine was tested for presence of blood and leukocytes using Multistix 9 Urinalysis Strips (Bayer, Elkhart, IN) and samples containing blood or leukocytes were excluded. Creatinine concentration of urine was determined using a commercial kit (Sigma, St. Louis, MO) according to the manufacturer's protocol. Protein concentration of urine was determined by the Bradford method using bovine serum albumin as the standard.

### Patient population.

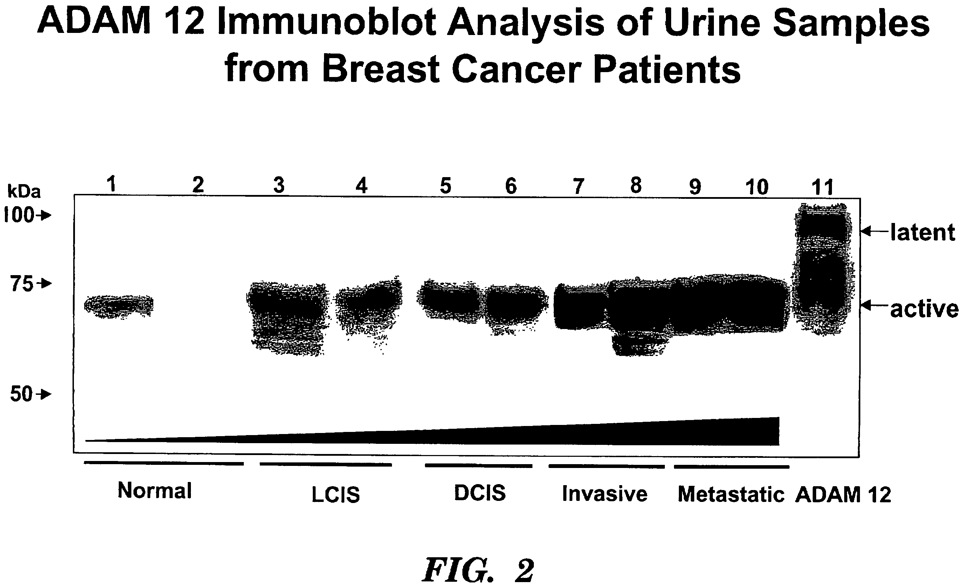

117 urine samples were analyzed for the presence of ADAM 12. The control group consisted of 46 women (median age ~50 years) with no discernable disease. The breast cancer group included 71 patients diagnosed with various stages of breast cancer including *atypical ductal hyperplasia (ADH), lobular carcinoma in situ* (*LCIS*), *ductal carcinoma in situ* (*DCIS*), locally *invasive breast cancer (IBC)* and metastatic disease (see Fig. 2).

### Western Blot Analysis.

Equal amounts of proteins (20 µg) were separated by SDS-PAGE under reducing conditions. Resolved proteins were electrophoretically transferred to nitrocellulose membranes (Schleicher & Schuell, Keene, NH) and treated as previously described (15). Labeled proteins were visualized with enhanced chemiluminescence (Pierce Chemical Company, Rockford, IL). Polyclonal antibodies against human ADAM 12, rb122 (4), S-18 (sc-16526, Santa Cruz Biotechnology, CA) were used at 1 µg/ml and 2 µg/ml concentration respectively. Band intensities were analyzed with UN-SCAN-IT™ (Silk Scientific, Orem, UT) software digitizer technology.

### Statistical Analysis.

Sensitivity, specificity, false-positive rate (FPR), false-negative rate (FNR) and likelihood ratio (LR) were calculated using standard formulas. Presence of ADAM 12 was compared between cancer groups (*ADH*/*LCIS, DCIS, IBC* and metastatic disease) and controls using logistic regression analysis. For each group, the presence of ADAM 12 is expressed as a percentage and 95% confidence intervals were calculated based on the normal approximation. The nonlinear relationship between ADAM 12 level and breast cancer was modeled using logistic regression analysis with probability determined by maximum likelihood estimation for selected intervals of ADAM 12 (17). Median levels of ADAM 12 were compared between controls and cancer patients using the Kruskal-Wallis test with pairwise comparisons based on Mann-Whitney U-tests whereas mean levels were compared by analysis of variance (ANOVA). In order to evaluate the diagnostic accuracy of ADAM 12, receiver operating characteristic (ROC) curve analysis was utilized to plot all pairs of true-positives and false-positives for varying levels of ADAM 12. ROC curves were constructed for differentiating between all breast cancer patients and controls and separately for each cancer group. ROC curves that go up steeply from (0,0) and reach near (0,1) indicate a test that is almost perfect in discriminating cancer patients from controls whereas curves close to the 45-degree diagonal line have low discriminatory power. Area under the curve (AUC) with 95% confidence intervals were determined using the Hanley-McNeil algorithm as an index of the performance of ADAM 12 as a biomarker of disease. In addition, the AUC equals the probability that a randomly chosen pair of patients and controls will be correctly classified. Actual numbers of cancer patients and controls are depicted as histograms with the theoretical probability curve superimposed to illustrate the increasing likelihood of breast cancer for higher levels of ADAM 12. To account for multiple comparisons among the five independent groups (four breast cancer groups according to stage of disease and the normal control group), a conservative two-tailed criterion of P<0.01 (0.05 divided by 5) was regarded as statistically significant. All statistical analysis was performed using the SAS statistical package (version 6.12, SAS Institute, Cary, NC). A power analysis indicated that a minimum of 13 patients in each of the breast cancer stages would provide 80% power for detecting differences in ADAM 12 level (version 5.0, nQuery Advisor, Statistical Solutions, Boston, MA).

### RESULTS

### Isolation and identification of ADAM 12.

Urine from breast cancer patients was subjected to stepwise chromatography and in an effort to isolate proteases an affinity chromatography step was performed using Gelatin Sepharose. The fact that ADAM 12 bound Gelatin Sepharose beads supported our data (see below) that this enzyme has gelatin-degrading capability. The fraction collected when 20% DMSO was applied to the Gelatin Sepharose column contained several distinct ~52, 80, 120 and 140 kDa protein bands (data not shown). MALDI-TOF analysis of the ~120 kDa protein band identified it as a disintegrin and metalloprotease domain 12 (ADAM 12) (accession NP_003465). Four distinct peptides spanning the amino acid sequence of human ADAM 12 were identified. The high MOWSE score, percentage of masses matched, and the number of peptides identified predicted the presence of ADAM 12 in the protein band analyzed with high confidence. To confirm these results, a larger cohort of urines including samples from breast cancer patients as well as age-matched, sex-matched controls was analyzed for ADAM 12. Urine samples were concentrated, normalized for protein (20 µg total protein was used for each analysis) and subjected to western blot analysis. In non-reduced urine samples, a polyclonal ADAM 12-specific antibody, rb122 recognized an ~120 kDa protein band (data not shown), whereas an -68 kDa band was detected when the sample was reduced with β-mercaptoethanol and boiled for 5 min. Immunoblot analysis with other ADAM 12-specific antibodies, polyclonal antibody S 18 and monoclonal antibodies 8F8 and 6E6 gave similar results (data not shown). Based on molecular weight the ~68 kDa band was identified as the mature form of ADAM 12-S (5). In light of the fact that the ADAM 12-specific antibody detected the mature form of the protein in urine of cancer patients the identification of two peptides from the prodomain of ADAM 12 by mass spectrometry was an interesting finding. It has been reported that the ADAM 12 prodomain remains associated with the protease even after furin cleavage (6) under non-reducing conditions. This suggests that tryptic cleavage (an essential step preceding mass spectrometric analysis) of this mature ADAM 12-associated propeptide resulted in the detection of the two peptides from the prodomain. Though the size of the protein band detected by immunoblot analysis using ADAM 12-specific antibody is most consistent with that of mature ADAM 12-S, the polyclonal antibody rb122 recognizes the cysteine-rich region of ADAM 12, a region of shared homology in both -L and -S forms. Therefore we cannot rule out the possibility that the band detected is a processed form of ADAM 12-L. Full-length ADAM 12-L has a reported mass of ~120 kDa whereas the mature (without the propeptide) membrane-anchored form is ~90 kDa (18). Though the ~120 kDa species analyzed by mass spectrometry contained the full-length protein, immunoblot analysis indicated that the major species in urine appears to be the ~68 kDa mature form of the enzyme.

### Substrate specificity of ADAM 12.

To test the ability of ADAM 12 to degrade ECM proteins, recombinant ADAM 12-S protein was expressed in *Cos*-7 cells and purified to homogeneity as described previously. When assayed by substrate zymography, ADAM 12 could proteolytically degrade gelatin. The purified human ADAM 12-S sample consisted of a ~68 kDa mature form of the enzyme that displayed a gelatinase activity band. ADAM 12 also degraded fibronectin and Type IV collagen but did not degrade casein when assayed by substrate zymography or Type I collagen when tested in the radiometric collagenase assay (data not shown). The gelatin-degrading activity of ADAM 12 was used to evaluate the effect of different classes of protease inhibitors on this enzyme. Pure ADAM 12-S was incubated with gelatin at 37 °C for either 2 or 18 h in the absence (Fig. 2C lane 3-4) or presence of various inhibitors. Disappearance of the ~140 kDa band indicated that ADAM 12 cleaved gelatin in a time-dependant manner. The classical chelators of zinc metalloproteases; EDTA and 1, 10-phenanthroline inhibited gelatin degradation by ADAM 12 whereas addition of 1 mM Marimastat resulted in partial inhibition of enzyme activity. The endogenous inhibitors TIMP-1, TIMP-2 and TIMP-4 did not inhibit the gelatinase activity of ADAM 12 even at concentrations 5-fold higher than the enzyme, however, TIMP-3 (500 nM) was an effective inhibitor of ADAM 12 activity.

### ADAM 12 levels during disease progression.

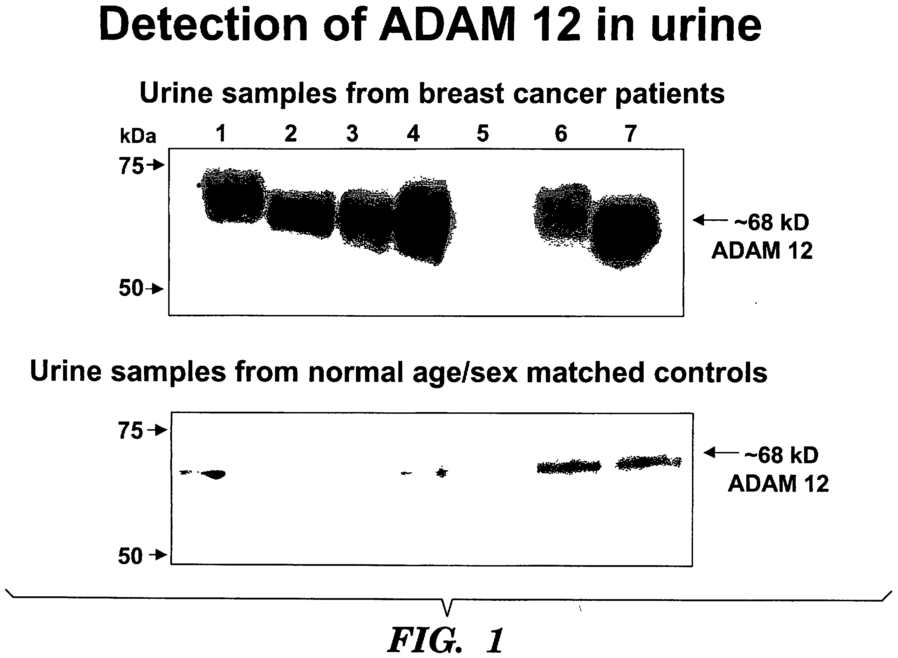

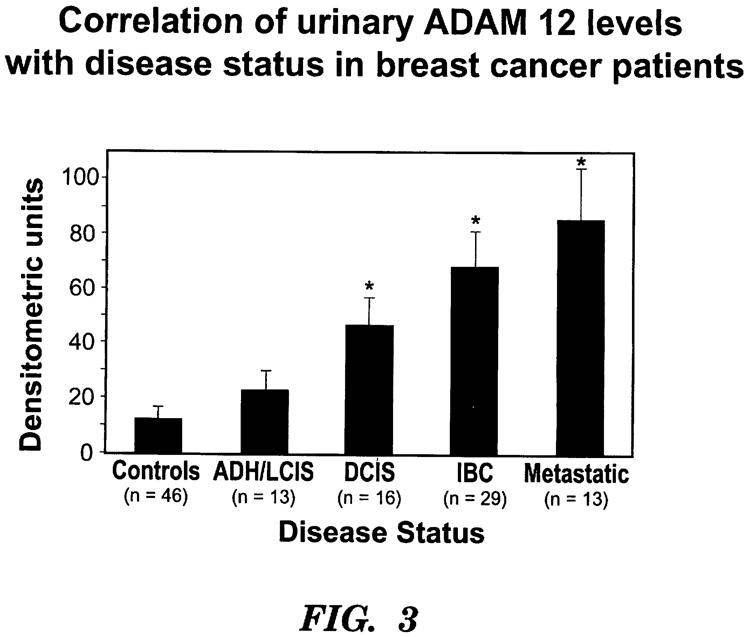

One hundred-seventeen urine specimens were analyzed, including 46 from healthy age-matched female volunteers and 71 from patients that had been diagnosed with various stages of breast cancer. The majority of samples from cancer patients (Fig. 1, Table I) were positive for ADAM 12 in contrast to the normal urines in which ADAM 12 was detected with significantly lower frequency. The frequency of detection of the ADAM 12 species was higher (94%) in the breast cancer group (67 out of 71 urines were positive) as compared to the control group (15%)(7 out of 46 were positive). Urine samples from the breast cancer patients and control group also differed in the levels of ADAM 12 protein. Immunoblot analysis of representative urine samples from healthy individuals and patients with progressive stages of breast cancer is presented in Fig. 2. Densitometric analysis of ADAM 12 bands indicated that levels of urinary ADAM 12 increase with progressive stage of disease. Very low or no ADAM 12 was detected in controls, slightly higher protein levels were associated with the initial stages of breast cancer e.g. *ADH*/*LCIS* and *DCIS* whereas significantly higher levels were observed in urine from patients with locally invasive and metastatic disease (Fig. 3).

### Statistical Analysis.

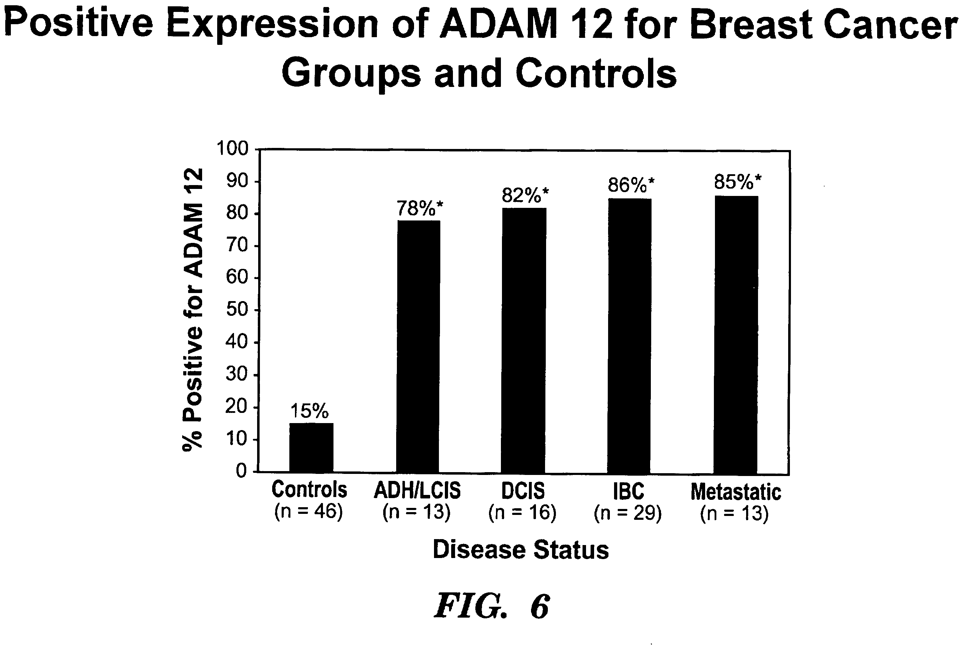

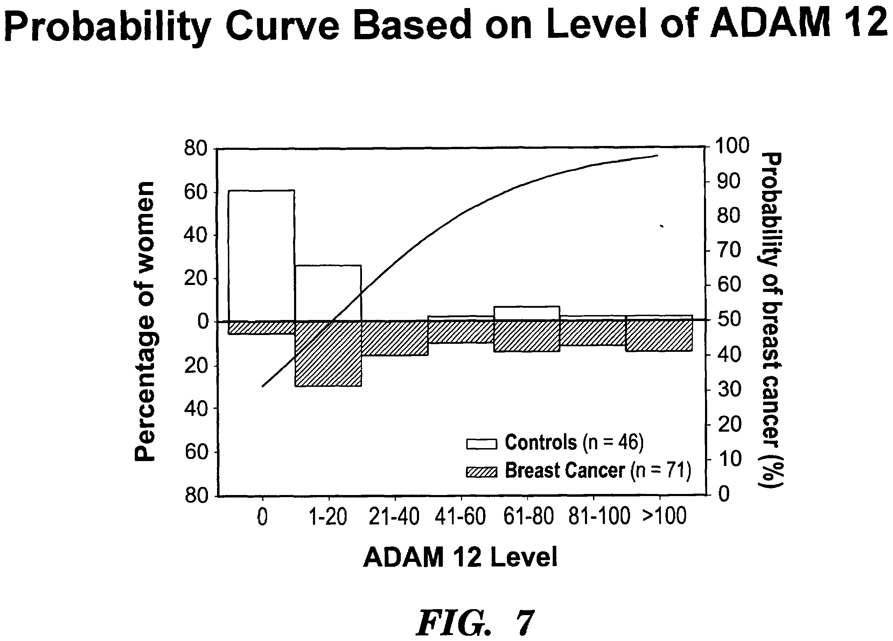

Table I presents the percentage of patients with positive expression of ADAM 12 as well as the median levels for each group. The mean densitometric score for control samples (12 DU) was set as threshold and % positive for each cancer group was calculated relative to the control group. As indicated in Figure 6, based on comparing percentages, there was a significantly higher proportion of individuals in each breast cancer group with expression of ADAM 12 compared to controls (all P < 0.01). Table II shows the diagnostic characteristics of ADAM 12 as a dichotomous test (based on positive or negative test result) for all patients and for each cancer stage group. For all cancer patients the sensitivity of ADAM 12 based on any level of positive expression is 0.94 (95% confidence interval: 0.86-0.98) and the specificity is 0.61 (28 of 46 controls) (95% confidence interval: 0.46-0.75). Setting a threshold value of 12 DU yielded a much higher specificity of 0.85 (39 of 46 controls) for all cancer patients. Overall accuracy was 0.81 based on 95 of the 117 individuals correctly classified as cases or controls (95% confidence interval: 0.73-0.88). The calculated likelihood ratio (LR) was 2.4 which for a positive test result indicates that presence of urinary ADAM 12 is associated with 2.4 times higher likelihood that the individual has breast cancer. Median levels of ADAM 12 for all 71 cancer patients and the 46 controls were 37 (interquartile range: 14-84) and 0 (interquartile range: 0-7), respectively (P < 0.001, Mann Whitney U-test). Receiver operating characteristic (ROC) curves were constructed for the various groups including all cancer patients, patients with *ADH*/*LCIS*, *DCIS*, *IBC* and metastatic disease to determine the relationship between the TPR (sensitivity) and FPR (1-specificity) for the empirical ADAM 12 levels as a continuous variable (data not shown). All ROC curves indicated excellent discrimination and rose sharply from the 45-degree diagonal line (i.e., the line of nondiscrimination). The ROC curves based on distinguishing between normal controls and women with IBC and those comparing controls and patients with metastatic disease had the highest AUC (0.90 and 0.92, respectively) for ADAM 12 levels greater than 0, the sensitivity for identifying patients with IBC and metastatic disease was perfect (i.e., sensitivity = 1.00). Logistic regression analysis was used to derive the probability of breast cancer based on ADAM 12 level (Fig. 7). The empirical levels of ADAM 12 are shown as histograms for cases and controls with the percentage of women having ADAM 12 levels represented on the x-axis. Among the 46 controls, 28 (61%) had ADAM 12 levels of 0 whereas only 4 of the 71 cancer patients (6%) had ADAM 12 levels of 0. In addition, 6 controls (13%) had ADAM 12 level above 20 whereas 46 of the 71 cancer patients (65%) had levels over 20. Logistic regression confirmed a highly significant direct relationship between ADAM 12 level and the probability of cancer (likelihood ratio test = 34.62, P < 0.0001). As indicated by the theoretical nonlinear curve in Figure 7, the probability of breast cancer is 32% for individuals having an ADAM 12 level of 0 (i.e. no expression) and nearly 50% when levels are between 1 and 20. The model predicts that for ADAM 12 levels between 21 and 40, the probability of breast cancer is 67%, 80% when levels are between 41 and 60, 90% for levels between 61 and 80, 95% for individuals having ADAM 12 levels of 81-100 and 98% when levels exceed 100.

**Table I. ADAM 12 Expression in Breast Cancer Patients and Controls**

| | | **Controls** | **ADH/LCIS** | **DCIS** | **IBC** | **Metastatic** |
|---|---|---|---|---|---|---|
| **No. patients** | | 46 | 13 | 16 | 29 | 13 |
| **Age, years** | | 41 ± 13 | 52 ± 6 | 57 ± 13 | 59 ± 12 | 55 ± 7 |
| **Presence of ADAM 12** | | 7 (15%) | 10 (78%) | 13 (82%) | 25 (86%) | 11 (85%) |
| **ADAM 12 Level (DU)** | **Mean ± SE** | 12.4 ± 4.2 | 23.0 ± 6.5 | 47.0 ± 9.8 | 68.4 ± 12.9 | 85.9 ± 17.8 |
| | **Median** | 0 | 14 | 46 | 43 | 80 |
| | **IQR** | 0-7 | 12-31 | 15-67 | 20-92 | 42-106 |
| | **Range** | 0-144 | 0-80 | 0-140 | 6-289 | 4-290 |

| | | | | | | |
|---|---|---|---|---|---|---|
| ADH = *atypical ductal hyperplasia*, LCIS = *lobular carcinoma in situ*, DCIS = *ductal carcinoma in situ*, IBC = *locally invasive breast cancer*, DU = densitometric units, IQR = interquartile range. | | | | | | |

**Table II. Diagnostic Characteristics of ADAM 12 in Differentiating Breast Cancer Patients and Controls**

| | **All Patients** | **ADH/LCIS** | **DCIS** | **IBC** | **Metastatic** |
|---|---|---|---|---|---|
| **Sensitivity** | 0.94 (67/71) | 0.85 (11/13) | 0.88 (14/16) | 1.00 (29/29) | 1.00 (13/13) |
| **FNR** | 0.06 (4/71) | 0.15 (2/13) | 0.12 (2/16) | 0.00 (0/29) | 0.00 (0/13) |
| **Specificity** | 0.61 (28/46) | 0.61 (28/46) | 0.61 (28/46) | 0.61 (28/46) | 0.61 (28/46) |
| **FPR** | 0.39 (18/46) | 0.39 (18/46) | 0.39 (18/46) | 0.39 (18/46) | 0.39 (18/46) |
| **Accuracy** | 0.81 (95/117) | 0.66 (39/59) | 0.68 (42/62) | 0.76 (57/75) | 0.70 (41/59) |
| **LR Positive Test** | 2.4 | 2.4 | 2.3 | 2.6 | 2.6 |

| | | | | | |
|---|---|---|---|---|---|
| ADH = *atypical ductal hyperplasia*, LCIS = *lobular carcinoma in situ*, DCIS = *ductal carcinoma in situ*, IBC = *locally invasive breast cancer*, FNR = false-negative rate, FPR = false-positive rate, LR = likelihood ratio. | | | | | |

### REFERENCES

1. Buxbaum, J. D., Liu, K. N., Luo, Y., Slack, J. L., Stocking, K. L., and Peschon, J. J. (1998) J. Biol. Chem. 273, 27765-27767
2. Chubinskaya, S., Mikhail, R., Deutsch, A., and Tindal, M. H. (2001) J. Histochem. Cytochem. 49, 1165-1176
3. Seals, D. F., and Courtneidge, S. A. (2003) Genes Dev. 17, 7-30
4. Gilpin, B. J., Loechel, F., Mattei, M. G., Engvall, E., Albrechtsen, R., and Wewer, U. M. (1998) J. Biol. Chem. 273, 157-166
5. Loechel, F., Gilpin, B. J., Engvall, E., Albrechtsen, R., and Wewer, U. M. (1998) J. Biol. Chem. 273, 16993-16997
6. Loechel, F., Fox, J. W., Murphy, G., Albrechtsen, R., and Wewer, U. M. (2000) Biochem. Biophys. Res. Commun. 278, 511-515
7. Shi, Z., Xu, W., Loechel, F, Wewer, U. M., and Murphy, L. J. (2000) J. Biol. Chem. 275, 18574-18580
8. Asakura, M., Kitakaze, M., Takashima, S., Liao, Y., Ishikura, F., Yoshinaka, T., Ohmoto, H., Node, K., Yoshino, K., Ishiguro, H., Asanuma, H., Sanada, S., Matsumara, Y., Takeda, H., Beppu, S., Tada, M., Hori, M., and Higashiyama, S. (2002) Nature Med., 8, 35-40
9. Iba, K., Albrechtsen, R., Gilpin, B. J., Loechel, F., and Wewer, U. M. (1999) Amer. J. Pathol. 154, 1489-1501
10. Iba, K., Albrechtsen, R., Gilpin, B., Frohlich, C., Loechel, F., Zolkiewska, A., Ishiguro, K., Kojima, T., Liu, W., Langford, J. K., Sanderson, R. D., Brakebusch, C., Fassler, R., and Wewer, U. M. (2000) J. Cell Biol. 149, 1143-1155
11. Le Pabic, H., Bonnier, D., Wewer, U. M., Coutand, A., Musso, O., Baffet, G., Clement, B., and Theret, N. (2003) Hepatology 37, 1056-1066
12. Lochter, A., Sternlicht, M. D., Werb, Z., and Bissell, M. J. (1998) Ann. N. Y. Acad. Sci. 857, 180-193
13. Kleiner, D. E., and Stetler-Stevenson, W. G. (1999) Cancer Chemother. Pharmacol. 43, S42-S51
14. Moses, M. A., Wiederschain, D., Loughlin, K. R., Zurakowski, D., Lamb, C. C., and Freeman, M. R. (1998) Cancer Res. 58, 1395-1399
15. Yan, L., Borregaard, N., Kjeldsen, L., and Moses, M. A. (2001) J. Biol. Chem. 276, 37258-37265
16. Moses, M. A., Sudhalter, J., and Langer, R. (1990) Science 248, 1408-1410
17. Breslow, N. E., and Day, N. E. (1980) in Statistical methods in cancer research. Vol. 1-The analysis of case-control studies pp. 192-224, Lyon, France: IARC
18. Cao, Y., Kang, Q., Zhao, Z. and Zolkiewska, A. (2002) J. Biol. Chem. 277, 26403-26411
19. Pieper, R., Gatlin, C. L., McGrath, A. M., Makusky, A. J., Mondal, M., Seonarain, M., Field, E., Schatz, C. R., Estock, M. A., Ahmed, N., Anderson, N. G., and Steiner, S. (2004) Proteomics 4, 1159-1174
20. Thongboonkerd, V., McLeish, K. R., Arthur, J. M., and Klein, J. B. (2002) Kidney Inter. 62, 1461-1469
21. Millichip, M. I., Dallas, D. J., Wu, E., Dale, S., and Mckie, N. (1998) Biochem. Biophys. Res. Comm. 245, 594-598
22. Martin, J., Eynstone, L. V., Davies, M., Williams, J. D. and Steadman, R. (2002) J. Biol. Chem. 277, 33683-33689
23. Shannon, J. D., Baramova, E. N., Bjarnason, J. B., and Fox, J. W. (1989) J. Biol. Chem. 264, 11575-11583
24. Alfandari, D., Cousin, H., Gaultier, A., Smith, K., White, J. M., Darribere, T., DeSimone, D. W. (2001) Curr. Biol. 11, 918-930
25. Amour, A., Knight, C. G., Webster, A., Slocombe, P. M., Stephens, P. E., Knauper, V., Docherty, A. J., and Murphy, G. (2000) FEBS Lett. 473, 275-279
26. Amour, A., Slocombe, P. M., Webster, A., Butler, M., Knight, C. G., Smith, B. J., Stephens, P. E., Shelley, C., Hutton, M., Knauper, V., Docherty, A. J., and Murphy, G. (1998) FEBS Lett. 435, 39-44
27. Zou, J., Zhu, F., Liu, J., Wang, W., Zhang, R., Garlisi, C. G., Liu, Y-H., Wang, S., Shah, H., Wan, Y., and Umland, S. P. (2004) J. Biol. Chem. 279, 9818-9830
28. Visse, R., and Nagase, H. (2003) Cir. Res. 92, 827-839
29. Fernández, C. A., Butterfield, C., Jackson, G., and Moses, M. A. (2003) J. Biol. Chem. 278, 40989-40995
30. Prestigiacomo, A. F., Lilja, H., Pettersson, K., Wolfert, R. L., and Stamey, T. A. (1996) J. Urol. 156,350-354
31. Moertel, C. G., Fleming, T. R., Macdonald, J. S., Haller, D. G., Laurie, J. A., and Tangen, C. (1993) J. Am. Med. Assoc. 270, 943-947
32. Berek, J. S., and Bast, R. C., Jr. (1995) Cancer (Phila.) 76, 2092-2096

## Claims

1. An *in vitro* method for diagnosing cancer of epithelial origin in a patient comprising:
a. measuring ADAM 12 levels present in a test sample obtained from the patient;
b. comparing the level of ADAM 12 in the test sample with the level of ADAM 12 present in a control sample;
wherein a higher level of ADAM 12 in the test sample as compared to the level of ADAM 12 in the control sample is indicative of cancer of epithelial origin; and
wherein said test sample is selected from the group consisting of blood and urine.

2. An *in vitro* method for prognostic evaluation of a patient suspected of having or having, cancer of epithelial origin comprising:
a. measuring the level of ADAM 12 present in a test sample obtained from the patient;
b. comparing the level determined in step (a) to a level of ADAM 12 in a control sample; and
c. evaluating the prognosis of said patient based on the comparison of step (b), wherein a level of ADAM 12 in the test sample that is at least 3 fold greater than the level of ADAM 12 in a control sample indicates an aggressive form of cancer and therefore a poor prognosis; and
wherein said test sample is selected from the group consisting of blood and urine.

3. The method of claim 1 or 2, wherein said test sample is urine.

4. The method of any one of the preceding claims, wherein the cancer of epithelial origin is selected from the group consisting of breast cancer, basal cell carcinoma, adenocarcinoma, gastrointestinal cancer, lip cancer, mouth cancer, esophageal cancer, small bowel cancer, stomach cancer, colon cancer, liver cancer, bladder cancer, pancreas cancer, ovary cancer, cervical cancer, lung cancer, skin cancer, prostate cancer, and renal cell carcinoma.

5. The method of claim 1, wherein the presence of ADAM 12 is detected using an antibody-based binding moiety which specifically binds ADAM 12.

6. The method of any of the preceding claims, wherein the level of ADAM 12 is measured by measuring the level of ADAM 12 protein.

7. The method of claim 6, wherein the level of ADAM 12 protein is measured by a method comprising the steps of :
a. contacting the test sample, or preparation thereof, with an antibody-based binding moiety which specifically binds ADAM 12 to form an antibody-ADAM 12 complex; and
b. detecting the presence of the complex, thereby measuring the level of ADAM 12 present.

8. The method according to claim 7, wherein the antibody-based binding moiety is labeled with a detectable label.

9. The method according to claim 8, wherein the label is selected from the group consisting of a radioactive label, a hapten label, a fluorescent label, and an enzymatic label.

10. The method according to claim 7, wherein the antibody-based binding moiety is an antibody.

11. The method according to claim 10, wherein the antibody is a monoclonal antibody.

12. Use of a kit comprising at least one antibody that specifically binds ADAM 12 as a means to detect ADAM 12 in a method of diagnosing cancer of epithelial origin in a test sample selected from blood and urine.

13. The use of a kit according to claim 12, wherein the kit comprises two antibodies that specifically bind to ADAM 12, one antibody is immobilized on a solid phase and one antibody is detectably labeled.

14. The use of a kit according to claim 12, wherein the kit further comprises directions for use.

## Patentansprüche

1. Ein *In vitro*-Verfahren zur Diagnose eines epithelialen Krebses in einem Patienten, umfassend:
a. Messen des ADAM-12-Proteingehalts in einer vom Patienten erhaltenen Probe;
b. Vergleich des ADAM-12-Gehalts in der Probe mit dem ADAM-12-Gehalt in einer Kontrollprobe;
wobei ein höherer ADAM-12-Gehalt in der Probe im Vergleich zum ADAM-12-Gehalt in der Kontrollprobe einen epithelialen Krebs anzeigt; und
wobei die Probe aus Blut und Urin besteht.

2. Ein *In vitro*-Verfahren zur prognostischen Bewertung eines Patienten, bei dem ein epithelialer Krebs vermutet wird oder der an einem epithelialen Krebs erkrankt ist, umfassend:
a. Messen des ADAM-12-Gehalts in einer vom Patienten erhaltenen Probe;
b. Vergleich des in Schritt (a) bestimmten Gehalts mit dem ADAM-12-Gehalt in einer Kontrollprobe;
c. Auswertung der Prognose des Patienten anhand dem Vergleich in Schritt (b), wobei ein ADAM-12-Gehalt in der Probe, der mindestens das 3-fache des ADAM-12-Gehalts in einer Kontrollprobe ausmacht, eine aggressive Form des Krebses und daher eine schlechte Prognose darstellt; und
wobei die Probe aus Blut und Urin besteht.

3. Das Verfahren gemäß Anspruch 1 oder 2, wobei die Probe Urin ist.

4. Das Verfahren gemäß einem der vorhergehenden Ansprüche, wobei es sich bei dem epithelialen Krebs um Brustkrebs oder Basalzellkarzinom, Adenokarzinom, Magen-Darm-Krebs, Lippenkrebs, Mundkrebs, Speiseröhrenkrebs, Dünndarmkrebs, Magenkrebs, Darmkrebs, Leberkrebs, Blasenkrebs, Bauchspeicheldrüsenkrebs, Eierstockkrebs, Gebärmutterhalskrebs, Lungenkrebs, Hautkrebs, Prostatakrebs und Nierenzellkarzinom handelt.

5. Das Verfahren gemäß Anspruch 1, wobei die Anwesenheit des ADAM-12-Proteins unter Verwendung einer auf Antikörper basierenden Bindungseinheit nachgewiesen wird, die insbesondere das ADAM-12-Protein bindet.

6. Das Verfahren gemäß einem der vorhergehenden Ansprüche, wobei der ADAM-12-Gehalt durch Messung des ADAM-12-Proteingehalts gemessen wird.

7. Das Verfahren gemäß Anspruch 6, wobei der ADAM-12-Proteingehalt durch ein Verfahren gemessen wird, das folgende Schritte umfasst:
a. Kontakt der Probe oder deren Herstellung mit einer Antikörper-basierten Bindungseinheit, die spezifisch ADAM 12 bindet und einen Antikörper-ADAM-12-Komplex bildet; und
b. Erfassen der Anwesenheit des Komplexes, wodurch der ADAM-12-Gehalt gemessen wird.

8. Das Verfahren gemäß Anspruch 7, wobei die auf Antikörper basierende Bindungseinheit mit einer nachweisbaren Markierung markiert wird.

9. Das Verfahren gemäß Anspruch 8, wobei die Markierung eine radioaktive Markierung, eine Haptenmarkierung, eine fluoreszierende Markierung oder eine enzymatische Markierung ist.

10. Das Verfahren gemäß Anspruch 7, wobei die auf Antikörper basierende Bindungseinheit ein Antikörper ist.

11. Das Verfahren gemäß Anspruch 10, wobei der Antikörper ein monoklonaler Antikörper ist.

12. Verwendung einer Ausrüstung in einem Verfahren zur Diagnose von epithelialen Krebserkrankungen bei einer aus Blut und Urin ausgewählten Probe, die mindestens einen spezifisch ADAM 12 bindenen Antikörper zum Nachweis von ADAM 12 umfasst.

13. Die Verwendung einer Ausrüstung gemäß Anspruch 12, wobei die Ausrüstung zwei Antikörper umfasst, die sich insbesondere an ADAM 12 anbinden, wobei ein Antikörper in einer festen Phase immobilisiert und ein Antikörper nachweisbar markiert wird.

14. Die Verwendung einer Ausrüstung gemäß Anspruch 12, wobei die Ausrüstung weiterhin eine Gebrauchsanweisung enthält.

## Revendications

1. Une méthode in vitro pour diagnostiquer le cancer d'origine épithéliale chez un patient comprenant :
a. mesurer les niveaux d'ADAM 12 présents dans un échantillon d'essai obtenu du patient ;
b. comparer le niveau d'ADAM 12 dans l'échantillon d'essai avec le niveau d'ADAM 12 présent dans l'échantillon témoin ;
dans laquelle un niveau élevé d'ADAM 12 dans l'échantillon d'essai tel que comparé au niveau d'ADAM 12 dans l'échantillon témoin est révélateur de cancer d'origine épithéliale ; et
dans laquelle ledit échantillon d'essai est sélectionné à partir du groupe composé de sang et d'urine.

2. Une méthode de diagnostic d'évaluation in vitro d'un patient soupçonné d'avoir ou ayant un cancer d'origine épithéliale comprenant :
a. mesurer le niveau d'ADAM 12 présent dans l'échantillon d'essai obtenu du patient ;
b. comparer le niveau déterminé à l'étape (a) à un niveau d'ADAM 12 dans un échantillon témoin ; et
c. évaluer le pronostic dudit patient basé sur la comparaison de l'étape (b), dans laquelle un niveau d'ADAM 12 dans un échantillon témoin qui est au moins 3 fois supérieur au niveau d'ADAM 12 dans un échantillon témoin indique une forme de cancer agressive et par conséquent un pauvre pronostic ; et
dans laquelle ledit échantillon d'essai est sélectionné à partir du groupe composé de sang et d'urine.

3. La méthode de la revendication 1 ou 2, dans laquelle ledit échantillon d'essai est de l'urine.

4. La méthode de l'une quelconque des revendications précédentes, dans laquelle le cancer d'origine épithéliale est sélectionné à partir du groupe composé du cancer du sein, du carcinome basocellulaire, d'adénocarcinome, du cancer gastrointestinal, du cancer des lèvres, du cancer de la bouche, du cancer de l'oesophage, du cancer de l'intestin grêle, du cancer de l'estomac, du cancer du côlon, du cancer du foie, du cancer de la vessie, du cancer du pancréas, du cancer des ovaires, du cancer du col de l'utérus, du cancer des poumons, du cancer de la peau, du cancer de la prostate et du carcinome rénale.

5. La méthode de la revendication 1, dans laquelle la présence d'ADAM 12 est détectée en utilisant une fraction de liaison basée sur les anticorps qui lie en particulier ADAM 12.

6. La méthode selon l'une quelconque des revendications précédentes, dans laquelle le niveau d'ADAM 12 est mesurée en mesurant le niveau de protéine d'ADAM 12.

7. La méthode de la revendication 6, dans laquelle le niveau de protéine d'ADAM 12 est mesuré par une méthode comprenant les étapes suivantes :
a. mettre en contact un échantillon d'essai, ou la préparation de celui-ci, avec une fraction de liaison basée sur les anticorps qui lie en particulier ADAM 12 pour former un ensemble anticorps-ADAM 12 ; et
b. détecter la présence de l'ensemble, mesurant ainsi le niveau d'ADAM 12 présent.

8. La méthode selon la revendication 7, dans laquelle la fraction de liaison basée sur les anticorps est étiquetée avec une étiquette détectable.

9. La méthode selon la revendication 8, dans laquelle l'étiquette est sélectionnée à partir du groupe composé d'une étiquette radioactive, une étiquette d'haptène, une étiquette fluorescente, et une étiquette enzymatique.

10. La méthode selon la revendication 7, dans laquelle la fraction de liaison basée sur les anticorps est un anticorps.

11. La méthode selon la revendication 10, dans laquelle l'anticorps est un anticorps monoclonal.

12. L'utilisation d'un kit comprenant au moins un anticorps qui se lie en particulier à ADAM 12 comme moyen de détecter ADAM 12 dans une méthode qui permet de diagnostiquer le cancer d'origine épithéliale dans un échantillon d'essai sélectionné à partir du sang et de l'urine.

13. L'utilisation d'un kit selon la revendication 12, dans laquelle le kit comprend deux anticorps qui se lient en particulier à ADAM 12, un anticorps immobilisé sur une phase solide et un anticorps étiqueté de façon détectable.

14. L'utilisation d'un kit selon la revendication 12, dans laquelle le kit comprend par ailleurs un mode d'emploi.
